# EUROPEAN PATENT APPLICATION

(11) **EP 4 342 974 A1**
(43) Date of publication of application: **27.03.2024**
(21) Application number: 23150494.5
(22) Date of filing: 05.01.2023
(51) Int. Cl.: C12M 3/00, C12M 1/00, C12N 15/87, C12M 1/36

(54) **INTEGRATED BIOPROCESSING SYSTEM FOR PERFORMING A BIOPROCESS ON A CELL CULTURE**

(30) Priority: 21.09.2022 WO PCT/IB2022/000491; 13.12.2022 WO PCT/EP2022/085651
(71) Applicant: The Automation Partnership (Cambridge) Ltd., Hertfordshire SG8 5WY (GB)
(72) Inventor: Stacey, Adrian, Hertfordshire SG8 5WY (GB); Prouté, Fanny, Hertfordshire SG8 5WY (GB)
(74) Representative: Gottschald Patentanwälte Partnerschaft mbB

(57) **Abstract**

The invention relates to an integrated bioprocessing system for performing a bioprocess on a cell culture, wherein the bioprocess comprises at least one unit operation, wherein the integrated bioprocessing system (1) comprises a cartridge (2) with a fluidic structure (3) for containing a liquid, in particular the cell culture, a drive module (4) for performing the unit operation in the fluidic structure (3) by driving the cartridge (2), wherein the cartridge (2) and the drive module (4) are individually selected according to the unit operation to be performed and operatively coupled with each other, several work sections (5), each work section (5) comprising a standardized interface (6) for operative coupling to the individually selected drive module (4), wherein each work section (5) is configured to perform the unit operation of the bioprocess by being operatively coupled to the drive module (4) and driving the drive module (4), and a coupling mechanism (7), which automatically couples the drive module (4) to one of the work sections (5) via the standardized interface (6).

## Description

The present invention relates to an integrated bioprocessing system for performing a bioprocess on a cell culture according to claim 1, to a cartridge with a fluidic structure for containing a liquid, in particular the cell culture, according to claim 15, to a drive module for performing a unit operation in a fluidic structure of a cartridge by driving the cartridge according to claim 16, to a method for performing a bioprocess on a cell culture with an integrated bioprocessing system according to claim 17 and to a use of a, in particular disposable, cartridge and/or a, in particular reusable, drive module according to claim 21.

The term "bioprocesses" comprises all kinds of processes in the broad field of biotechnology, like in the area of cell and gene therapy including allogenic or autologous production of genetically modified immune cells. Bioprocesses are often related to or performed with different kinds of cell cultures, which are, for example, taken from a patient, generated, modified and/or used, in particular to obtain a specific desired product. The cell cultures themselves may be the desired product or may be used to produce the desired product respectively in further processes. For example, if a respective product shall be obtained, a certain cell culture may be generated and modified such that the product is produced and obtained thereafter. Generally, different kinds of cell cultures may lead to different kinds of products. However, in context with this application, the term "bioprocess" has to be understood broadly such that it comprises partial processes, like generation or modification of cell cultures or usage of cell cultures to obtain the product, and entire processes on cell cultures likewise.

During the performance of bioprocesses, one or more "unit operations" are being performed. Unit operations are basic process steps for reaching a certain physical and/or chemical change of the cell culture. Examples for common unit operations are enrichment, selection, activation and genetic modification.

Since the bioprocess to be performed is, inter alia, very dependent on the cell culture itself and cell cultures are very heterogeneous regarding their composition, process flexibility is very relevant when performing bioprocesses on different cell cultures.

For example, in the field of cell and gene therapy, bioprocesses on liquid immune cell cultures are performed, wherein the liquid immune cell cultures may be used as starting material. This starting material may be quite heterogeneous regarding its composition, exemplarily because each patient is in a different condition, such that several parameters of the used cell cultures may vary, like the type and concentration of different cell types, the overall cell viability and vitality and/or the amount and type of impurities within the cell culture. For that reason, the bioprocess and particularly the unit operation or unit operations to be performed are highly dependent on the cell cultures such that these have to be adjusted accordingly.

For that reason, in recent years, bioprocesses are more and more performed on integrated bioprocessing systems. Such integrated bioprocessing systems allow simultaneous performance of several bioprocesses on different cell cultures, wherein the bioprocesses or rather the unit operation or unit operations to be performed can be adjusted accordingly to the respective cell culture. Here, the quite heterogeneous parameters and properties of the different cell cultures can be considered accordingly such that different bioprocesses on different cell cultures may be performed with the same integrated bioprocessing system.

Besides that, integrated bioprocessing systems provide a certain degree of automation, which enables performing the bioprocesses largely without user interaction. This can lead to a reduction of costs of the overall process, higher redundancy in view of process robustness and process reliability as well as process optimization, for example with regard to a contamination risk of the cell cultures. Since, the bioprocesses are often cost-extensive, highly individual and moreover complex, it can be seen, for which reasons the utilization of integrated bioprocessing systems is advantageous.

An example of a known integrated bioprocessing system for performing a bioprocess on a cell culture, wherein the bioprocess comprises at least one unit operation, is disclosed in WO 2021/212124 A1. Here, the integrated bioprocessing system comprises a rack with different modules, wherein each module comprises an instrument to perform a certain unit operation. The rack can be assembled by a user as needed, in particular regarding the instruments within the different modules, for example when the integrated bioprocessing system is configured. A cartridge with a fluidic structure, which contains the cell culture, is connected sequentially to the different kinds of instruments to perform the desired unit operation within one of the modules. The connection of the cartridge to the respective instrument is performed one after another automatically by a robotic arm.

Although the known integrated bioprocessing system provides in general an already flexible and automatized solution for performing several bioprocesses on different cell cultures, there is still a need for further improvement of integrated bioprocessing systems regarding flexibility and automatization.

For example, the known integrated bioprocessing system may be adjusted concerning the different modules, when assembled. Here, the user may arrange the different instruments, which are mandatory for performing the different unit operations, in the modules as needed. When assembling the integrated bioprocessing system accordingly the demand of the different modules or rather instruments within the modules has to be estimated. For example, if a certain unit operation is expected to be performed more often, modules with instruments for performing this unit operation should be in a superior number in contrast to modules with instruments for performing another unit operation, which is expected to be performed less often. However, the adjustment cannot be changed individually during the bioprocesses such that a considerable number of modules may not be used and the overall capacity of the integrated bioprocessing unit decreases. The adjustment of the modules may indeed be performed by a user, for example by changing the instruments in certain modules, but involves extensive modification work, which may lead to time delays and additional costs.

For the beforehand-mentioned reasons, the invention is based on the problem of providing an integrated bioprocessing system, which is further optimized with regard to flexibility and automatization.

The above-noted object is solved by the features of claim 1.

The main realization of the present invention is that an integrated bioprocessing system is provided, which utilizes a cartridge according to a unit operation, which is going to be performed and that the cartridge can be used at any of several work sections flexibly. The cartridge is adjusted to perform the respective unit operation. In contrast to the known system, the unit operation can be performed with the cartridge at any of several work sections without modification work, such that in particular any work section at which currently no unit operation is performed, may be used for performing the unit operation. To realize this feature, the integrated bioprocessing system comprises a drive module, which is separately designed to fit the cartridge and the work sections and which is selected according to the respective unit operation and cartridge. The drive module may be designed correspondingly to the respective cartridge. Furthermore, the drive module may be selected as needed out of a group of different drive modules. The drive module drives the cartridge, when operatively coupled to each other such that the unit operation can be performed, whereat the drive module itself is operatively coupled to one of the work sections via a standardized interface, which is comprised by each of the work sections. The standardized interface makes it possible to operatively couple the drive module to any of the several work sections. Since, the cartridge and the drive module are coupled, and the drive module is coupled to one of the work sections, the drive module may act like an adapter to utilize the cartridge at any of the work sections.

For providing a high degree of automation, the operatively coupling of the drive module to the work section is performed automatically by a coupling mechanism such that no user interaction is needed. By providing the standardized interface, the operative coupling can be easily realized since the coupling itself may be standardized and thus can be performed by the coupling mechanism.

In detail, an integrated bioprocessing system for performing a bioprocess on a cell culture is proposed, wherein the bioprocess comprises at least one unit operation, wherein the integrated bioprocessing system comprises a cartridge with a fluidic structure for containing a liquid, in particular the cell culture,
a drive module for performing the unit operation in the fluidic structure by driving the cartridge, wherein the cartridge and the drive module are individually selected according to the unit operation to be performed and operatively coupled with each other, several work sections, each work section comprising a standardized interface for operative coupling to the individually selected drive module, wherein each work section is configured to perform the unit operation of the bioprocess by being operatively coupled to the drive module and driving the drive module, and a coupling mechanism, which automatically couples the drive module to one of the work sections via the standardized interface.

According to a preferred embodiment of claim 2, the integrated bioprocessing system comprises several cartridges and several drive modules. With a number of cartridges and drive modules, it is possible to perform several bioprocesses with the integrated bioprocessing system. The bioprocesses may be performed on different cartridges operatively coupled to the different drive modules, which are in turn are each operatively coupled to one of the work sections. However, the bioprocesses may be performed on a single cell culture, in particular one after another, and/or on several cell cultures. It is possible, to perform the bioprocesses at least partly simultaneously and/or sequentially.

Claim 3 further specifies the features of the integrated bioprocessing system regarding the fluid transfer from a cartridge, in particular to another cartridge. An automatic fluid transfer enables using different cartridges for a given bioprocess. Hence, the flexibility and automation of the integrated bioprocessing system are greatly increased.

The advantageous embodiment according to claim 4 is directed to features regarding a sequence of different unit operations of the bioprocess to be performed. Hereby, a possibility to sequentially perform different unit operations on the cell culture may be provided. By using individual adjusted cartridges for the unit operations to be performed, a highly flexible and moreover highly efficient integrated bioprocessing system may be provided. It is particularly preferred that different unit operations of the sequence are performed on different cartridges such that each unit operation can be performed on individually adjusted cartridges. However, it is also possible that more than one unit operation, for example two unit operations, are performed on the same cartridge and a following unit operation is performed on another cartridge.

According to a preferred embodiment of claim 5, the cartridge is designed as a disposable, in particular driveless, cartridge and/or the drive module is designed as a reusable drive module. Thus, the fluid contacting part, namely the fluidic structure of the cartridge, is a single-use product, which is used for a single cell culture only for preventing contamination. However, the drive module, which is not contacted by the cell culture, can be used several times to drive different cartridges such that efficiency of the integrated bioprocessing system can be improved. By making the cartridge a single use component, the integrated bioprocessing system allows relatively cheap performance of bioprocesses even for as highly individual bioprocesses as those performed on immune cell cultures. At the same time, by using the drive module as an adapter between the work sections and the cartridge, it becomes possible to provide updated or completely different unit operations with just an update to the drive module and the cartridges.

Claim 6 provides advantageous embodiments of the integrated bioprocessing system regarding an engagement point, which enables transfer of liquid from and to the cartridge. In particular, if the cartridge comprises a tube engagement point, wherein for example a tube can be connected by tube welding, a sterile fluid connection between the cartridge and another cartridge or a receptacle may be provided.

In claim 7, the fluidic structure and the drive module are further specified. For performing the unit operation, the fluidic structure comprises one or more functional devices. For driving the cartridge, the drive module comprises a drive structure, wherein the drive structure comprises one or more drive devices for driving the one or more functional devices. Since the fluidic structure, in particular the functional device or functional devices, may be designed driveless and thus need to be driven to perform the unit operation, the provided embodiment provides a solution for individually driving the individual functional device or devices by the individual drive device or drive devices. The drive device or the drive devices can be adjusted, in particular when the drive module is fabricated, according to the functional device or functional devices to be driven.

As specified in claim 8, it is preferred that the cartridge comprises at least one cartridge sensor for sensing physical and/or chemical properties of the liquid. Alternatively or additionally, the drive module comprises at least one module sensor for sensing properties of the cartridge and/or the drive module and/or the liquid. By sensing different properties, in particular when performing the unit operation, the condition of the liquid, like the temperature, pressure, pH, etc., as well as the unit operation itself may be monitored. With monitoring certain properties, it may be possible, to adjust the unit operation as needed with regard to the monitored data.

Furthermore, claim 9 specifies that the fluidic structure, and thus the cartridge, comprises one or more cartridge containers and/or one or more fluid transfer elements. Hereby, the cartridge can be designed more independently with regard to components, which may be needed for the unit operation to be performed, and for transfer within the cartridge. The cartridge may provide an overall solution for at least partly performing the unit operation, since, for example, components can be comprised in the cartridge container or cartridge containers and/or fluid can be transferred within the different parts of the cartridge by the fluid transfer element or fluid transfer elements. It is particularly preferred that the fluid transfer elements are designed as active fluid transfer elements, such as pumps or valves, and that these active fluid transfer elements are actuated by an interface actuator of the standardized interface. Thus, the active fluid transfer elements, which may be part of several cartridges for performing several unit operations, can be directly actuated by the standardized interface such that the drive modules may be simplified.

The preferred embodiment according to claim 10 is directed to features regarding the spatial design of the cartridge. In particular, if the cartridge comprises two or more adaptable partitions with one or more partition elements, the cartridges may be adapted for individual use as needed more easily, for example by a user or automatically. A housing may protect the parts of the cartridge and may prevent damage or contamination from outer impact. A frame may provide easier handling of the cartridge.

Claim 11 further specifies the integrated bioprocessing system regarding the work sections, wherein the integrated bioprocessing system comprises a worktop, on which the standardized interfaces of the work sections are arranged. Thus, a space-saving embodiment may be provided.

Moreover, it is preferred according to claim 12 that the standardized interfaces each comprise an electrical interface, a mechanical interface, a data interface, an optical interface and/or a fluidic interface. Here, a flexible solution of a standardized interface is provided, which provides an extensive range of functionality such that all kinds of unit operations may be performed.

An advantageous embodiment according to claim 13 is directed to features of the coupling mechanism. For individual and flexible handling, in particular coupling, of the cartridges and/or drive modules inside the integrated bioprocessing system, the coupling mechanism may comprise a robotic mechanism and/or a conveyor mechanism. It is possible that the robotic mechanism and the conveyor mechanism interact such that, for example, the conveyor mechanism transports the cartridge and/or the drive mechanism to a certain location inside the integrated bioprocessing system and the robotic mechanism picks the cartridge and/or the drive module from this location, for example, for operative coupling of the drive module to one of the work sections. For simultaneously handling of more than one cartridge and/or more than one drive module, the coupling mechanism may comprise more than one robotic mechanism and/or conveyor mechanism.

Claim 14 specifies a further preferred embodiment, which provides an accurate solution for interaction of the robotic mechanism and the cartridge, the drive module and the standardized interfaces and/or the worktop. By providing robot engagement point of the cartridge and the drive module as well as robot anchor points of the standardized interfaces and/or the worktop, the robot mechanism can easily operatively couple the cartridge and the drive module and the drive module and the work sections.

Another teaching according to claim 15, which is of equal importance, relates to a cartridge with a fluidic structure for containing a liquid, in particular the cell culture, designed such that the cartridge can be used in an integrated bioprocessing system according to any of the proposed embodiments.

All explanations and features given with regard to the integrated bioprocessing system are fully applicable to the proposed cartridge and vice versa.

Another teaching according to claim 16, which is of equal importance, relates to a drive module for performing a unit operation in a fluidic structure of a cartridge by driving the cartridge designed such that the drive module can be used in an integrated bioprocessing system according to any of the proposed embodiments.

All explanations and features given with regard to the integrated bioprocessing system and the cartridge are fully applicable to the proposed drive module and vice versa.

Another teaching according to claim 17, which is of equal importance, relates to a method for performing a bioprocess on a cell culture with an integrated bioprocessing system, wherein the bioprocess comprises at least one unit operation and, in particular, wherein the integrated bioprocessing system is designed according to any of the proposed embodiments, wherein the method comprises individually selecting a cartridge and a drive module according to the unit operation to be performed and operatively coupling the cartridge and the drive module with each other, wherein a coupling mechanism automatically operatively couples the drive module to one of several work sections via a standardized interface of the respective work section, wherein the work section, which is operatively coupled to the drive module, drives the drive module and the drive module drives the cartridge such that the unit operation is performed in the fluidic structure.

All explanations and features given with regard to the integrated bioprocessing system, the cartridge and the drive module are fully applicable to the proposed method and vice versa.

According to claim 18, it is preferred that the proposed method is partially or completely performed automatically. Thus, the overall process of performing the bioprocess on the cell culture with the integrated bioprocessing system may be furthermore automated and therefore may be further improved regarding efficiency aspects, like cost-reduction, time-optimization, etc.

Claim 19 is directed to the operative coupling of the cartridge and the drive module as well as the operative coupling of the drive module and one of the work sections. It is proposed that the operatively coupling of the cartridge and the drive module may be performed before coupling of the drive module to the respective work sections such that the cartridge and the drive module may be handled as a unit within the integrated bioprocessing system, in particular when operatively coupling the drive module and the respective work section. It is alternatively proposed that the operatively coupling of the cartridge and the drive module may be performed after coupling of the drive module to the respective work sections such that the cartridge may be handled independently of the drive module. Furthermore, it is alternatively proposed that the operative couplings are performed at least partially simultaneously such that the process may be further optimized, in particular regarding time and/or flexibility.

Furthermore, claim 20 provides a solution for performing several bioprocesses on several cell cultures in parallel such that the efficiency of performing the method with the integrated bioprocessing system can be improved.

Another teaching according to claim 21, which is of equal importance, relates to a use of a, in particular disposable, cartridge and/or a, in particular reusable, drive module in an integrated bioprocessing system according to any of the proposed embodiments.

All explanations given with regard to the integrated bioprocessing system, the cartridge, the drive module and the method are fully applicable to the proposed use and vice versa.

In the following, embodiments of the invention are explained with respect to the drawing. The drawing shows in
- Fig. 1,: a schematic presentation of an embodiment of a proposed integrated bioprocessing system in perspective view,
- Fig. 2,: a schematic presentation of the integrated bioprocessing system according to Fig. 1 in top view and
- Fig. 3,: a schematic presentation in top view of respectively a) a standardized interface of a work sections ; b) a drive module; c) a consumable.

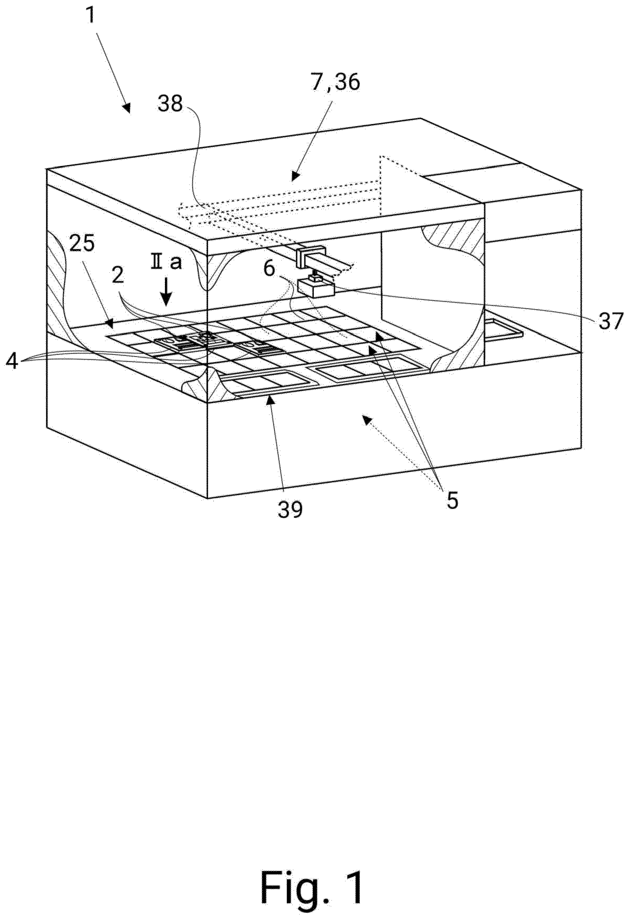

Provided is an integrated bioprocessing system 1 for performing a bioprocess on a cell culture, as exemplarily shown in Fig. 1. Here and preferably, the integrated bioprocessing system 1 is designed as a closed system, wherein the inner parts of the integrated bioprocessing system 1 may be reached via accesses, if needed.

The bioprocess to be performed comprises at least one unit operation. The integrated bioprocessing system 1 comprises a cartridge 2 with a fluidic structure 3 for containing a liquid, in particular the cell culture. The integrated bioprocessing system 1 furthermore comprises a drive module 4 for performing the unit operation in the fluidic structure 3 by driving the cartridge 2. The cell culture may be preferably a liquid immune or naive cell culture. The bioprocess may be started with an initial cell culture.

The integrated bioprocessing system 1 shown in the drawings is preferably adapted to perform a bioprocess for the manufacturing of genetically modified T cells. Here, T cells are genetically modified to express a chimeric antigen receptor (CAR). Consequently, the term "CAR-T cells" describes T cells that have been genetically modified to express a CAR. The genetically modified CAR-T cells, which represent the product of the bioprocess, may be administered to a patient and used to start or resume cancer treatment in the patient. As the bioprocess is performed, the initial immune cell culture is gradually processed. All explanations given are mainly directed to such a bioprocess. It may be pointed out, however, that those explanations are fully applicable to other bioprocesses as well.

The term "liquid immune cell culture" is to be understood in a broad sense and refers to an immune cell culture comprising at least one type of immune cells suspended as particles in any type of liquid. As will be explained below, the liquid immune cell culture may comprise other cell types that are not immune cells. Hence, the term "liquid immune cell culture" refers to a liquid immune cell culture at any stage during the bioprocess. Consequently, the type and fraction of immune cells present in the liquid immune cell culture will change during the bioprocess applied as certain immune cells are enriched or depleted from the liquid immune cell culture and/or the immune cells are genetically modified.

The term "immune cells" generally refers to different types of white blood cells. Hence, the term "immune cells" includes a variety of cells, for example, but not limited to dendritic cells, T lymphocytes, also referred to as T cells, B lymphocytes, natural killer cells, macrophages or the like. Immune cells may also include subtypes of immune cells, for example tumor-infiltrating lymphocytes or different types of T cells. Subtypes of a certain type of immune cells may be classified based on the type of antigen present at the cell surface. Hence, the term immune cells may for example refer to T cells comprising the surface antigen CD4 ("CD4+ T cells"). Typically, a certain type of immune cells, e.g., T cells, preferably a certain subtype of immune cells, e.g., CD4+ T cells, will be selectively enriched by the bioprocess, while other immune cells, e. g. macrophages, and/or other cell types that are not immune cells, e. g., erythrocytes, and/or other subtypes of immune cells, e.g., CD8+ T cells, will be depleted from the liquid immune cell culture. The immune cells to be enriched are referred to as target immune cells, all other components to be depleted from the liquid immune cell culture are referred to "impurities". Further, and as mentioned above, the target immune cells might be genetically modified.

The term "naive cells" refers to cells that can still differentiate into different target cell types. In particular, stem cells and their derivatives prior to full differentiation into a specific cell type are naive cells. The term also comprises naive immune cells.

The term "liquid" is to be understood in a broad sense as well and refers to any liquid and/or particle-containing liquid that is processed within the integrated bioprocessing system 1. Hence, the term liquid might refer to media, waste, the liquid immune cell culture, byproducts obtained during the bioprocess, samples and/or an initial immune cell culture.

Preferably, an initial immune cell culture is obtained in a process called "leukapharesis". In leukapharesis, immune cells are obtained from the patient. Additionally or alternatively, the initial immune cell culture might also be obtained from a tissue of the patient. The initial immune cell culture might also be obtained by one or more donors that are not the patient.

Depending on the source of the initial cell culture and the bioprocess to be carried out, the initial cell culture, particularly the type, amount and distribution of impurities as well as target immune or naive cells might vary.

Presently, it is preferred that any initial immune cell culture used in the bioprocess is used for only a single patient or a small number of patients, for example up to ten patients. If that is not the case then the bioprocess presently described will yield only a cell culture for a single patient or a small number of patients and cell cultures for other patients are derived from different bioprocesses. Therefore, the proposed integrated bioprocessing system 1 is used for small scale bioprocesses.

The terms "drive" or "driving" should be understood broadly. Thus, drive or driving comprise all kind of actuation, which is mandatory to perform the unit operation. This in particular includes mechanical driving. For example, mechanical driving can be performed by a centrifuge drive, which drives a centrifuge head, and/or by a pump drive, which drives a pump head. The driving may also comprise a contactless energy transmission by electrical fields, magnetic fields or acoustic waves for example. It should be understood that the drive module 4 comprises some kind of driving for the cartridge 2 that influences the unit operation in some manner. Here an preferably, the driving is controlled by sensor feedback. Preferably, the actuation is dependent on sensor values concerning the cartridge, in particular measured inside the cartridge as will be explained below. It may be the case that an actuation is terminated when a sensor value indicates that the actuation has reached its target. More complex controlling is also possible. For example, a pump may be actuated until a sensor at a tube detects that the fluid has passed the sensor position.

Within the proposed integrated bioprocessing system 1, the cell culture on which the bioprocess is going to be performed is preferably comprised in the cartridge 2, in particular in the fluidic structure 3, wherein the drive module 4 is strictly separated therefrom. The cartridge 2 and the drive module 4 are separately designed, which may result in cost-benefits, since the cartridge 2 may be used one-time, whereas the drive module 4 may be used several times. Preferably, only the fluidic structure 3 may be potentially contacted by the liquid, in particular the cell culture or reagents or the like, wherein the drive module 4 may not be contacted. The cartridge 2 may comprise disposable parts, which are mandatory for performing the unit operation, and the drive module 4 may comprise reusable parts, which are mandatory for performing the unit operation.

The cartridge 2 and the drive module 4 are individually selected according to the unit operation to be performed. As described beforehand, several unit operations may be performed with the integrated bioprocessing system 1. The unit operations, which are being performed, may be different unit operations or of the same kind. To perform the unit operation as needed on the individual cell culture, the cartridge 2, which is accordingly adjusted to perform the unit operation as needed, has to be selected. The cartridge 2 may be taken from a group of differently adjusted cartridges 2. The same applies for the drive module 4, which may be adapted to drive the individually selected cartridge 2.

In the preferred embodiment the integrated bioprocessing system 1 comprises cartridges 2 and drive modules 4 that can be individually selected to perform a number of different bioprocesses. If the owner of the integrated bioprocessing system 1 wishes to add compatibility for another bioprocess, they may simply buy another type of drive module 4 and accompanying cartridges 2. Due to the standardization of the work sections 5, the integrated bioprocessing system 1 can flexibly adapt to new unit operations.

The cartridge 2 and the drive module 4 are operatively, in particular detachably, coupled with each other. Operative coupling of the cartridge 2 and the drive module 4 enables the drive module 4 to drive the cartridge 2. Since, the cartridge 2 may be driveless, this operative coupling is mandatory. It is preferred that the operative coupling of the cartridge 2 and the drive module 4 is reversible such that the cartridge 2 and the drive module 4 may be detached after performing the at least one unit operation.

The integrated bioprocessing system 1 moreover comprises several work sections 5. At each of the work sections 5, one or more of the unit operations may be performed. Thus, the integrated bioprocessing system 1 may be especially flexible regarding the location for performing unit operations.

However, to provide such flexibility and because the different unit operations require different cartridges 2 and thus different drive modules 4, each work section 5 comprises a standardized interface 6 for operative coupling to the individually selected drive module 4, wherein each work section 5 is configured to perform the unit operation of the bioprocess by being operatively, in particular detachably, coupled to the drive module 4 and driving the drive module 4. "Standardized" in this context means that different kinds of drive modules 4, which are individually adjusted according to the unit operation to be performed, are coupleable to these standardized interfaces 6. Since each work section 5 comprises the standardized interface 6, each or at least a few of the individually selected drive modules 4 may be operative coupled to these work sections 5 optionally such that different unit operations can be performed flexibly at different work sections 5. The unit operation to be performed may thus be not linked to an individual work section 5. It is preferred that the operative coupling of the drive module 4 to one of the work sections 5 is reversible such that the drive module 4 may be detached after performing the at least one unit operation.

For operative coupling of the drive module 4 to one of the work sections 5, the integrated bioprocessing system 1 comprises a coupling mechanism 7. The coupling mechanism 7 automatically couples the drive module 4 to one of the work sections 5 via the respective standardized interface 6 of the work section 5. By automatic operative coupling, the overall performance of the bioprocess may be further automatized. Preferably, the coupling mechanism 7 is designed to also automatically decouple the drive module 4 of the respective work section 5.

According to one embodiment it is proposed, that the integrated bioprocessing system 1 comprises several cartridges 2 each with a fluidic structure 3 for containing a liquid, in particular a cell culture. The several cartridges 2 may be used to perform several unit operations on the same cell culture or on different cell cultures. The unit operations may be similar unit operations and/or different unit operations, wherein the respective cartridges 2 may be similarly and/or differently adjusted accordingly. The unit operations may be performed sequentially, namely one after another, and/or at least partially simultaneously.

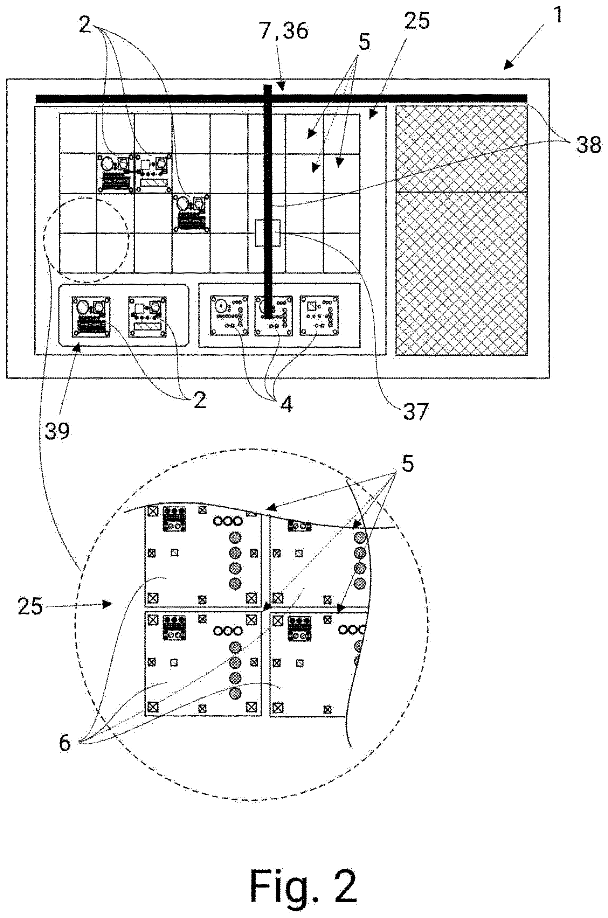

Preferably, the integrated bioprocessing system 1 comprises several drive modules 4 each for performing at least one unit operation in one of the fluidic structures 3 by driving one of the cartridges 2. For driving the several cartridges 2, several drive modules 4 are likewise utilized. However, it is possible that a part of the drive modules 4 are sequentially used for different of the several cartridges 2. The cartridges 2 and the drive modules 4 are each individually selected according to the unit operation to be performed and respectively operatively coupled with each other. Preferably, at least one of the cartridges 2 and one of the drive modules 4 are respectively operatively coupled such that the respective drive module 4 can drive the respective cartridge 2. However, at least some of the cartridges 2 and/or at least some of the drive modules 4 may be stored temporarily in a storage of the integrated bioprocessing system 1, as exemplarily shown in Fig. 2.

It is preferred that at least two drive modules 4 are each automatically coupled to one of the work sections 5 via the standardized interface 6 by the coupling mechanism 7. The work sections 5, on which the two drive modules 4 are coupled may be different work sections 5.

The integrated bioprocessing system 1 of Figs. 1 and 2 comprises several, exemplarily three, different cartridges 2, which each are operatively coupled to a respective drive module 4. With each of the cartridges 2, a individually selected unit operation can be performed on a cell culture. The unit operations are performed as needed. Here, the unit operations are partly simultaneously performed. The drive modules 4 are respectively each operatively coupled to one of the work sections 5 of the integrated bioprocessing system 1. As it can exemplarily be seen, the location, where the respective unit operation is performed, can be chosen, since the work sections 5 each comprise a standardized interface 6. The different drive modules 4 are, here and preferably, compatible with every one of the standardized interfaces 6 such that every combination of drive module 4 and cartridge 2 may be couplable to every work section 5. As it is exemplarily shown in Fig. 2, the integrated bioprocessing system 1 furthermore comprises several cartridges 2 as well as several drive modules 4 in a storage.

It is preferred that the fluidic structure 3 of the cartridge 2 contains liquid, in particular the cell culture. Particularly during the unit operation to be performed, the fluidic structure 3 may contain the liquid, such as a reagent or the cell culture itself, and thus is contacted by the liquid. However, the drive module 4 may not be contacted and thus contamination of the liquid, in particular the cell culture, is prevented. The at least one unit operation is performed on the liquid in the fluidic structure 3.

Preferably, after performing of the at least one unit operation, the fluidic structure 3 is fluidically connected to a fluidic structure 3 of another cartridge 2 and the liquid is transferred between the fluidically connected fluidic structures 3 of the cartridges 2. Since the cartridges 2 are preferably designed to perform a single unit operation or a small number of unit operations, but not the overall bioprocess on the cell culture, the liquid, in particular the cell culture, is preferably transferred to another cartridge 2 for performing another unit operation. The cartridges 2 being used preferably build a group of cartridges 2, wherein the group of cartridges 2 comprises the different cartridges 2 for respectively performing the unit operations of the overall bioprocess. The group of cartridges 2 may comprise further cartridges 2, wherein the liquid, in particular the cell culture, is transferred sequently to the different cartridges 2 and at least one unit operation is being performed on each cartridge 2 accordingly.

As exemplarily shown in Fig. 2, two of the cartridges 2 are arranged on neighboring work sections 5. Here, the cell culture, on which in the left-sided cartridge 2 a unit operation was performed, is transferred to the right-sided cartridge 2 to perform a consecutive unit operation. Therefore, the fluidic structures 3 of the two cartridges 2 are fluidically connected.

It is further preferred that the bioprocess to be performed on the cell culture comprises a sequence of unit operations. It is preferred that the sequence of unit operations is performed with at least two different cartridges 2. Furthermore and preferably, for driving the individually selected cartridges 2, the sequence of unit operations is performed with at least two different drive modules 4. The drive modules 4 may be each individually selected according to the unit operation of the sequence of unit operations to be performed. It is preferred that during the sequence of unit operations, in particular among two consecutive unit operations, liquid, in particular the cell culture, is transferred between two of the cartridges 2. Thus, one unit operation on the cell culture can be performed on one of the cartridges 2 and another consecutive unit operation can be performed on another of the cartridges 2.

In Fig. 2, it is exemplarily shown that the sequence of different unit operations is performed with two cartridges 2. As described beforehand, one unit operation is performed on the left-sided cartridge 2 and another unit operation is performed on the right-sided cartridge 2 after transfer of the cell culture. The cartridges 2 are adjusted as needed to perform the desired unit operation.

In the following, preferred embodiments of the integrated bioprocessing system 1 with regard to the features of the cartridge 2 and the drive module 4 are made. It has to be pointed out that all features and explanations are given for the cartridge 2 and the drive module 4 can also apply in case of several cartridges 2 and several drive modules 4 for each of the cartridges 2 and each of the drive modules 4 accordingly. Here, the further cartridges 2 and/or drive modules 4 may be designed similarly, in particular with regard to their outer shape, or differently, in particular with regard to their fluidic structure 3, which is designed accordingly to the unit operation to be performed, or partly similarly.

According to another preferred embodiment, the cartridge 2 is designed as a disposable cartridge 2. Since the cartridge 2, in particular the fluidic structure 3 of the cartridge 2, is potentially contacted by the liquid and in particular the cell culture, a specific cartridge 2 can be one-time used for a specific cell culture. The disposable cartridge 2 may comprise all single-use parts which are mandatory for performing the unit operation. Parts, which may be reusable for several unit operations, may be not comprised by the cartridge 2. It is additionally or alternatively preferred that the cartridge 2 is designed driveless. Preferably, the drive module 4 is designed as a reusable drive module 4. By strictly separating the fluid-contacted parts and the not fluid-contacted drive parts, the drive module 4 may be reused with different cartridges 2 to perform a certain unit operation several times on different cell cultures. The cartridge 2 and/or the drive module 4 can be standardized.

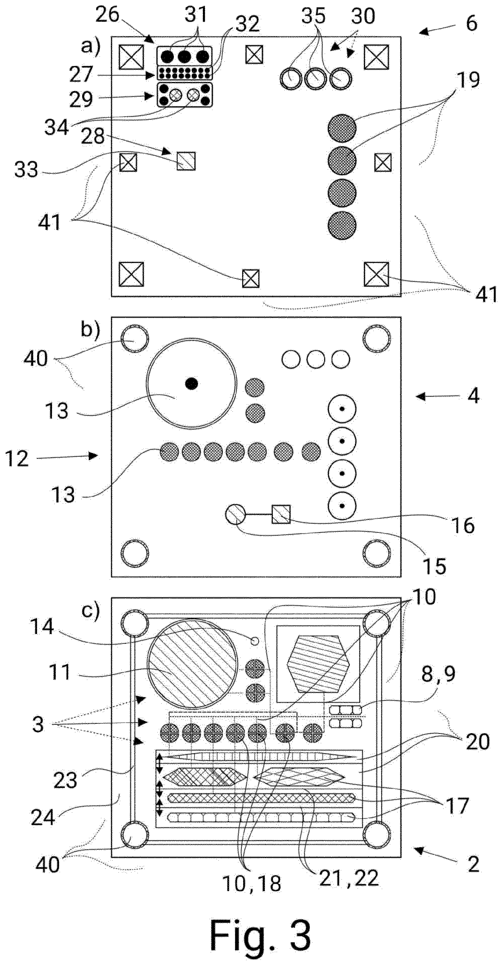

Exemplarily, Fig. 3c) shows a disposable cartridge 2. Here, all parts, which are potentially contacted by the liquid, in particular the cell culture, are comprised by the cartridge 2. Thus, the cartridge 2 is single-used with the specific cell culture to perform the specific unit operation and is afterwards disposed. As it can be seen and preferably, the cartridge 2 is driveless. Fig. 3b) shows a corresponding reusable drive module 4.

It is preferred, that the cartridge 2 comprises an engagement point 8 for fluidically connecting the fluidic structure 3 of the cartridge 2 to another fluidic structure 3 of another cartridge 2 such that the liquid, in particular the cell culture, can be transferred between the fluidically connected fluidic structures 3 of the cartridges 2. The engagement point 8 may provide access to the fluidic structure 3 of the cartridge 2 and may be used for inflow and outflow of the liquid from and to the fluidic structure 3 of the cartridge 2. Alternatively or additionally, the engagement point 8 may be utilized for fluidically connecting the fluidic structure 3 of the cartridge 2 to a fluidic structure 3 of a receptacle. A receptacle may comprise a bag for particularly storing, transporting and generally handling of the transferred liquid, in particular the transferred cell culture, between unit operations. Advantageously, the engagement point 8 is or the engagement points 8 each are designed as a tube engagement point 9. Here, it is preferred that a tube is connectable to the tube engagement points 9 to provide a fluidic connection between the cartridges 2, in particular by tube welding. The tube may be an, in particular external, tube or may be a fluid transfer element 10 of the cartridge 2, in particular a tube. Preferably, connection of the tube engagement points 9, in particular of the tubes of the tube engagement points 9, is enabled by tube welding. In Fig. 3c) a cartridge 2 with the tube engagement point 9 is exemplarily shown.

According to another preferred embodiment it is proposed, that the fluidic structure 3 comprises one or more functional devices 11 for at least partially performing the unit operation. The functional device 11 or functional devices 11 each serve to at least partially perform the unit operation, when driven by the drive module 4. Preferably, the functional device 11 or functional devices 11 may be designed as a heating element, a separation element, a selection element, a genetic modification element and/or a pump. It is preferred that the functional element or the functional elements is or are designed as a centrifuge chamber, a, in particular magnetic, separator chamber, an acoustic chamber, a flow cell, a washing chamber, a media exchange chamber and/or an electroporation chamber. The functional device 11 or the functional devices 11 may be, in particular potentially, contacted by the liquid, in particular the cell culture, when the unit operation is being performed. The functional device 11 or the functional devices 11 are preferably designed driveless and are each drivable by the corresponding drive structure 12, in particular a corresponding drive device 13, of the drive module 4. The liquid, in particular the cell culture, may be directly in contact of the functional device 11 during the performance of the unit operation.

It is further preferred that the drive module 4 comprises a drive structure 12 for driving the cartridge 2. Preferably the drive structure 12 comprises one or more drive devices 13 for driving the one or more functional devices 11. The drive device 13 or drive devices 13 are designed correspondingly to the respective functional device 11 of the cartridge 2. Preferably, the drive device 13 or drive devices 13 of the drive module 4 are designed as a centrifuge drive, a, in particular magnetic, separator drive, an acoustic drive, a flow cell drive, a washing drive, a media exchange drive and/or an electroporation drive.

Here and preferably, the functional device 11 acts directly on the liquid, in particular by contacting the liquid or by applying, possibly contactless, energy to the liquid in a way that directly influences the liquid, for example by providing a magnetic field for magnetic beads.

Advantageously, the cartridge 2 comprises at least one cartridge sensor 14 for sensing properties of the liquid, in particular of the cell culture. Properties may be cell culture characteristics like cell size and/or cell morphology and/or properties of the liquid like pH, temperature, dissolved oxygen concentration, or the like. The cartridge sensor 14 may be designed as a contactless sensor or as a contacting sensor, which directly contacting the liquid, in particular the cell culture. Preferably, the cartridge sensor 14 is designed as a disposable cartridge sensor 14 and thus is single-used only with a specific cell culture. The cartridge sensor 14 may be a temperature sensor, a pH sensor or a pressure sensor. Alternatively or additionally, the drive module 4 comprises at least one module sensor 15 for sensing properties of the cartridge 2 and/or the drive module 4 and/or the properties of the liquid, in particular of the cell culture. The module sensor 15 may be designed as a contactless sensor, in particular when sensing properties of the liquid. Here and preferably, the module sensor 15 is designed as a reusable sensor, which may be used multiple times with different cartridges 2.

It is furthermore preferred that the drive module 4 comprises a sensor control unit 16 for controlling the at least one cartridge sensor 14 and/or the at least one module sensor 15. The sensor control unit 16 may process the signals of the cartridge sensor 14 and/or the module sensor 15 such that the signals can be transferred via the standardized interface 6. The sensor control unit 16 may be used for calibration of the cartridge sensor 14 and/or the module sensor 15 and/or for pre-processing and/or recording of the according signals. The sensor control unit 16 may translate the sensor measurements into a standardized communication protocol used by or behind the standardized interface 6.

According to one embodiment it is proposed, that the fluidic structure 3 comprises one or more cartridge containers 17. The cartridge container 17 or the cartridge containers 17 may be designed as a reagent bag, a waste bag, a product bag, a storage bag and/or an incubation bag. The cartridge container 17 or the cartridge containers 17 may be designed to receive solids, liquids and gases. Within the cartridge container 17 or the cartridge containers 17 components, such as reagents, which are needed for the unit operation to be performed, may be contained. It is also possible that the cartridge container 17 or the cartridge containers 17 can receive waste, which accumulates during the unit operation. Furthermore, it is possible that the cartridge container 17 or the cartridge containers 17 can receive liquids, such as the cell culture itself, for incubation. It is particularly possible that the cartridge 2 comprises several containers with respective different of the foregoing named purposes. Preferably, the one or more cartridge containers 17 is or are each designed as a bag.

In Fig. 3c), for example, the cartridge 2 comprises several cartridge containers 17, namely a cartridge container 17 for waste, several cartridge containers 17 for reagents and cartridge containers 17 for storage of a liquid, such as the cell culture. Here, the containers are designed as bags such that the volume of the respective container changes correspondingly to the contained volume of liquid.

Additionally or alternatively, the cartridge 2 comprises one or more fluid transfer elements 10. Fluid transfer elements 10 allow to transfer fluid within the respective cartridge 2. Here and preferably, the one or more of the fluid transfer elements 10 is or are designed as a tube. With tubes, fluid, in particular the liquid contained in the fluidic structure 3, can be guided from and/or to the functional device 11 or functional devices 11 and/or cartridge container 17 or cartridge containers 17. The tube or the tubes may be designed as flexible and/or rigid tubes, such as pipes. Several tubes may be arranged within the cartridge 2 as a tube set. It is preferred that one or more of the fluid transfer elements 10 is or are designed as active fluid transfer elements 18. Active fluid transfer elements 18 may be valves and/or pump heads, whereas a valve enables controlling of fluid flow, in particular in a tube and a pump head enables pumping fluid from and/or to the functional device 11 or functional devices 11 and/or the cartridge container 17 or cartridge containers 17.

Possibly, the one or more active fluid transfer elements 18 is or are each operable by an interface actuator 19 of the standardized interface 6. Alternatively or additionally, the one or more fluid transfer elements 10 is or are each operable by one or more of the drive devices 13.

It is interesting to note that the standardized interface 6 may comprise one or more actuators driving the cartridge 2 directly bypassing the drive module 4. This makes sense in particular for the active fluid transfer elements 18 which may be recurring in many or all cartridges 2. The drive modules 4, of which there are preferably more than work section 5, thereby become cheaper. It is also obvious that there may be more cartridges 2 than drive modules 4. The drive module 4 may contain holes at the respective positions.

Also shown exemplarily in Fig. 3c), the cartridge 2 comprises several fluid transfer elements 10. Here, a part of the fluid transfer elements 10 is designed as tubes, whereby the tubes connect the parts of the cartridge 2 among each other. For controlling the different fluid flows, another part of the fluid transfer elements 10 is designed as active fluid transfer elements 18, such as valves and pump heads. The valves enable controlling of the different fluid flows, the pump heads enable pumping the respective fluids. For example, if a reagent from one of the cartridge containers 17 shall be pumped to another cartridge container 17, the pump heads as well as the valves are actuated such that the reagent can be guided from the one cartridge container 17 to the other.

According to another embodiment, the cartridge 2 comprises two or more partitions 20, which are formed by one or more partition elements 21. Preferably, the one or more partition elements 21 is or are designed such that the partitions 20 are adaptable, in particular with regard to the size of the respective partition 20. The partition 20 or the partitions 20 may be adaptable by moving and fixating the partition element 21 or partition elements 21. This may be performed during the adjustment, for example during fabrication, of the cartridge 2. It is preferred that the partition element 21 or partition elements 21 is or are each designed as a, particularly movable, partition wall 22.

Preferably, the cartridge 2 comprises a frame 23, wherein in particular the fluidic structure 3 is surrounded by the frame 23. It is furthermore preferred that the cartridge 2 comprises a housing 24, wherein the fluidic structure 3, in particular the one or more functional devices 11 and/or the one or more fluid transfer elements 10 and/or the one or more cartridge containers 17, and/or at least a part of the frame 23 is or are included in the housing 24.

As it can be exemplarily seen in Fig. 3c), the cartridge 2 comprises several partitions 20, which are displayed in the lower part of Fig 3c). The partitions 20 are formed by several partition elements 21, which are each designed as partition walls 22. Within each partition 20, one of the cartridge containers 17 is arranged. The partition elements 21 are partly arranged horizontally and partly arranged vertically according to Fig 3c). However, with regard to the integrated bioprocessing system 1 the partition elements 21 are arranged horizontally, as shown in Fig. 1. The partition elements 21 are moveable mounted such that they can be adapted according to the size of the contained cartridge container 17. As it can furthermore be seen and preferably, the cartridge 2 comprises the housing 24, wherein the fluidic structure 3 of the cartridge 2 is fully arranged inside the housing 24. The fluidic structure 3 is thus accessible by the engagement point 8 only.

According to another embodiment it is proposed, that the integrated bioprocessing system 1 comprises a worktop 25, wherein the standardized interfaces 6 of the work sections 5 are arranged on the worktop 25. The worktop 25 may be designed as a plane worktop 25 with a consistent level. It is preferred that the worktop 25 is arranged horizontally. Regarding the arrangement of the standardized interfaces 6, it has proven beneficial, if the standardized interfaces 6 are arranged in a cross direction and in a longitudinal direction of the worktop 25. It is preferred that the standardized interfaces 6 are evenly spaced in the cross direction and/or in the longitudinal direction of the worktop 25.

As it can be seen exemplarily in Figs. 1 and 2, the integrated bioprocessing system 1 comprises the horizontally arranged worktop 25. The worktop 25 arranges, here and preferably, the standardized interfaces 6 within the same level such that the different standardized interfaces 6 are easily accessible, in particular when operative coupling of the drive module 4 with one of the work sections 5 is performed. Furthermore, the standardized interfaces 6 arranged in a horizontal and a vertical row, as it can be seen in particular in Fig. 2. The standardized interfaces 6 of the several work sections 5 thus build a grid, which results in the integrated bioprocessing system 1 being space-saving.

Advantageously, the standardized interface 6 of one or more work sections 5 each comprises different kinds of interfaces, such as an electrical interface 26, a data interface 27, a mechanical interface 28, an optical interface 29 and/or a fluidic interface 30. The electrical interface 26 of each standardized interface 6 may enable electric powering of the drive module 4 and possibly the cartridge 2. Preferably, each of the electrical interfaces 26 comprise at least one electrical connector 31, particularly at least one power supply. The power supply or power supplies may be designed as a voltage source or voltage sources, in particular such that the voltage source provides ground, namely 0V, 5V and/or 20V.

The data interface 27 of each standardized interface 6 may enable data transfer from and/or to the cartridge 2 and/or the drive module 4, which is operatively coupled to one of the work sections 5, and/or in particular the sensor control unit 16. The data interface 27 of each standardized interface 6 is preferably used to control the cartridge 2 regarding the unit operation to be performed and/or the drive module 4 regarding the drive of the cartridge 2 and/or to control the unit operation itself. For example, the unit operation is controlled, in particular in real-time, regarding unit operation parameters, such as time, temperature, pressure, etc. The data interface 27 of each standardized interface 6 preferably comprises at least one data connector 32, which may be designed as a digital connector for transferring digital data and/or an analogue connector for transferring analogue signals. The mechanical interface 28 of each standardized interface 6 enables mechanic powering of the drive module 4, for example rotational power or the like. Preferably, the mechanical interface 28 comprise at least one mechanical connector 33. The optical interface 29 of each standardized interface 6 enables to transfer optical signals from and/to the cartridge 2 and/or the drive module 4, for example light signals or the like. The optical interface 29 of each standardized interface 6 may comprise at least one optical connector 34. The fluidic interface 30 of each standardized interface 6 may enable providing fluid from and/or to the cartridge 2, in particular fluids which are utilized during the unit operation. Here, preferably, fluids like liquid and/or gas is provided, such as water, cell culture medium, oxygen, nitrogen, carbon dioxide and/or inert gas. Furthermore, it is possible, that fluid is provided to power the drive module 4. It is preferred that the fluidic interface 30 of each standardized interface 6 comprises one or more fluidic connectors 35, in particular a gas supply and/or a liquid supply.

As a further preferred embodiment it is provided that the work sections 5 are designed each as standardized work sections 5 with the standardized interface 6. While the standardized interface 6 enables flexible coupling of at least a part of different drive modules 4, preferably all kinds of different drive modules 4, the standardized work sections 5 enable to simplify the general structure of the integrated bioprocessing system 1.

Regarding the embodiment of the coupling mechanism 7, the coupling mechanism 7 may comprise one or more robotic mechanisms 36. The robotic mechanism 36 or each of the robotic mechanisms 36 may comprise a robotic handling mechanism 37 for handling, in particular picking and releasing, the cartridge 2 and/or the drive module 4, and a robotic moving mechanism 38 for moving the robotic handling mechanism 37. Preferably, the robotic handling mechanism 37 performs coupling of the cartridge 2 and the drive module 4 and/or of the drive module 4 and the work sections 5 by the standardized interface 6. The robotic handling mechanism 37 can be designed such that it can pick and release the cartridge 2 and/or the drive module 4, also if both are already coupled. The robotic handling mechanism 37 may be designed as a gripper. The robotic moving mechanism 38 may move the robotic handling mechanism 37. The robotic moving mechanism 38 preferably is designed as a rail robotic moving mechanism 38, which comprises different moving axis or axes for linear movement in one, two or three dimensions, or as a robotic arm.

Additionally or alternatively, the coupling mechanism 7 comprises one or more conveyor mechanisms 39 for moving the cartridge 2 and/or the drive module 4 within the integrated bioprocessing system 1. The conveyor mechanism 39 or the conveyor mechanisms 39 is or are each designed such that the cartridge 2 and/or the drive module 4 can be moved inside the integrated bioprocessing system 1, in particular in two dimensional directions. It is preferred that at least one conveyor mechanism 39 is designed such that the cartridge 2 and/or the drive module 4 can be moved in elevational direction, for example such that the cartridge 2 and/or the drive module 4 is elevated from a lower stack, which may be located under the worktop 25, by the conveyor mechanism 39. The conveyor mechanism 39 may comprise one or more conveyors.

In Fig. 1 and 2 the coupling mechanism 7 of the integrated bioprocessing system 1 is exemplarily shown. The coupling mechanism 7 comprises the robotic mechanism 36 with the robotic handling mechanism 37 and the robotic moving mechanism 38. The robotic moving mechanism 38 is designed as a rail robotic moving mechanism 38, which provides three different moving axes for linear movement in each dimension. The moving axes of the robotic moving mechanism 38 in the horizontal dimensions can be seen in Fig. 2, for example. Furthermore, the coupling mechanism 7 of the exemplary embodiment comprises a not further shown conveyor mechanism 39 to elevate the cartridges 2 and the drive modules 4 in the stacks.

According to another embodiment of the invention it is proposed, that the cartridge 2 comprises one or more, in particular four, robot engagement points 40. The robot engagement points 40 may be designed as apertures, in particular such that the robot handling mechanism 37 can pick the cartridge 2. Preferably, the robot engagement points 40 of the cartridge 2 are arranged at outer edges of the frame 23, more preferably that the robot engagement point 40 or robot engagement points 40 is or are included in the housing 24.

Furthermore, it is preferred that the drive module 4 comprises one or more, in particular four, robot engagement points 40, wherein the robot engagement points 40 may be designed as apertures, in particular such that the robot handling mechanism 37 can pick the drive module 4. Preferably, the robot engagement points 40 of the cartridge 2 are arranged at outer edges of the drive module 4.

Here and preferably, the standardized interfaces 6 of the work sections 5 each comprise and/or the worktop 25 comprises robot anchor points 41. Preferably, the standardized interfaces 6 each comprise four, in particular eight, robot anchor points 41. It is preferred that the respective robot anchor points 41 restrict the respective standardized interface 6, particularly against neighboring standardized interfaces 6.

It is further preferred that at least a part of the robot anchor points 41 align with at least a part of the robot engagement points 40 when the cartridge 2 and the drive module 4 are coupled to the work section 5. This way, the cartridge 2 and/or the drive module 4 is held in its position. Thus, the robot engagement point 40 fulfill a double-function. Accordingly, the robot anchor points 41 may be designed as pins.

Another teaching which is of equal importance relates to a cartridge 2 with a fluidic structure 3 for containing a liquid, in particular the cell culture. The cartridge 2 is designed such that the cartridge 2 can be used in an integrated bioprocessing system 1 according to one of the proposed embodiments.

In Fig. 3c), an example of the cartridge 2 is shown. The cartridge 2 comprise the fluidic structure 3. The fluidic structure 3 is designed to contain liquid, in particular the cell culture. Preferably, the fluidic structure 3 contains all liquids, solids and/or gases, which are mandatory for the unit operation to be performed, in particular all reagents.

Preferably and shown in Fig. 3c), the cartridge 2 is designed driveless. The cartridge 2 thus have to be driven by an external drive, such as a drive module 4. Preferably, the cartridge 2 is couplable to the drive module 4. The cartridge 2 does preferably comprise all components for performing the unit operation to be performed, when driven by the drive module 4.

Here and preferably, the fluidic structure 3 of the cartridge 2 comprises the functional device 11, which is exemplarily a centrifuge chamber. The cartridge 2 may comprise several functional devices 11. The functional device 11 or the functional devices 11 can in particular be designed as a, in particular magnetic, separator chamber, a washing chamber, a media exchange chamber and/or an electroporation chamber. The functional device 11 or the functional devices 11 are potentially contacted with the liquid, in particular the cell culture, when the unit operation is performed.

Advantageously and shown in Fig. 3c), the fluidic structure 3 of the cartridge 2 comprises fluid transfer elements 10. Preferably, a part of the fluid transfer elements 10 is designed as tubes and another part of the fluid transfer elements 10 is designed as active fluid transfer elements 18, namely as valves and pump heads. Whereas the valves are not shown in Fig. 3c), the fluidic structure 3 of the embodiment in Fig. 3c) comprise nine pump heads, which enable different fluid flows, in particular liquid flows, within the cartridge 2. The active fluid transfer elements 18 may be actuated by drive devices 13 of a coupled drive module 4 and/or by interface actuators 19 of a standardized interface 6.

It is preferred and shown in Fig. 3c) that the cartridge 2 comprises several cartridge containers 17, which can contain and/or receive liquid, solid and/or gas. In the Fig. 3c) and preferably, the cartridge 2 comprises cartridge containers 17 which are designed as a reagent bag, a waste bag, a product bag, a storage bag and an incubation bag.

It is preferred that the cartridge 2, at least the fluidic structure 3, is sterilizable, for example by using chemical- and/or light- and/or temperature-sterilization. It is preferred that at least the fluidic structure 3, in particular the cartridge 2, is at least partly, preferably completely, out of plastic material.

Furthermore, it is preferred that the fluidic structure 3 is preassembled in the cartridge 2 such that, for example, the respective tubes are associated with the pump heads. The cartridge 2 may be sterilized and placed in a sterilized packaging prior to being inserted into the integrated bioprocessing system 1.

Another teaching which is of equal importance relates to a drive module 4 for performing a unit operation in a fluidic structure 3 of a cartridge 2 by driving the cartridge 2. The drive module 4 is designed such that the drive module 4 can be used in an integrated bioprocessing system 1 according to one of the described embodiments. The proposed drive module 4 can in particular be utilized with the beforehand described proposed cartridge 2.

An example of the drive module 4 is shown in Fig. 3b). Here and preferably, the drive module 4 is designed without a fluidic structure 3 such that the drive module 4 is not contacted by any fluids of the bioprocesses, which are performed.

Advantageously, the drive module 4 is designed such that it is couplable to a cartridge 2 and a work section 5 via an, in particular standardized, interface.

Here and preferably, the drive module 4 is designed as a handleable unit, which in particular can be handled independently of any work sections 5 and cartridges 2.

It is preferred and shown in Fig. 3b) that the drive module 4 comprises a drive structure 12. The drive structure 12 comprises several drive devices 13. The drive devices 13 are designed correspondingly to the functional devices 11 of a cartridge 2, which is going to be driven by the drive module 4. Here and preferably, the drive device 13 or drive devices 13 of the drive module 4 are designed as a centrifuge drive, a, in particular magnetic, cell separator drive, a cell washing drive, a media exchange drive and/or an electroporation drive.

Another teaching which is of equal importance relates to a method for performing a bioprocess on a cell culture with an integrated bioprocessing system 1, wherein the bioprocess comprises at least one unit operation and, in particular, wherein the integrated bioprocessing system 1 is designed according to one of the proposed embodiments. The method comprises individually selecting a cartridge 2 and a drive module 4 according to the unit operation to be performed and operatively coupling the cartridge 2 and the drive module 4 with each other, wherein a coupling mechanism 7 automatically operatively couples the drive module 4 to one of several work sections 5 via a standardized interface 6 of the respective work section 5, wherein the work section 5, which is operatively coupled to the drive module 4, drives the drive module 4 and the drive module 4 drives the cartridge 2 such that the unit operation is performed in the fluidic structure 3.

"Operatively coupled" or "operatively coupling" in context with this application may comprise coupling of the respective parts such that the parts are not only arranged to each other but rather the functions of the respective parts can be utilized as intended.

According to one embodiment it is proposed, that the method is performed at least in parts, in particular in total, automatically. It is preferred that the selection of the cartridge 2 and the drive module 4 according to the unit operation to be performed, the operative coupling and decoupling of the cartridge 2 and the drive module 4, the operative coupling and decoupling of the drive module 4 to one of the work sections 5, the fluidic coupling and decoupling of two of the cartridges 2 and/or the transfer of liquid between the two cartridges 2 can be performed automatically. Preferably, the method is, at least partially, automatically performed in response to an initiating computer command and/or a user command. More preferably, the operative coupling of the consumable module and the drive module 4 is performed automatically, preferably at least partially, in particular in total, by the coupling mechanism 7.

According to another embodiment it is proposed, that the cartridge 2 and the drive module 4 are operatively coupled before operative coupling of the drive module 4 to the respective work section 5 by the standardized interface 6. Alternatively it is possible that the cartridge 2 and the drive module 4 are operatively coupled after operative coupling of the drive module 4 to the respective work section 5 by the standardized interface 6. Alternatively, it is also possible that the cartridge 2 and the drive module 4 are operatively coupled at least partially simultaneously to operational coupling of the drive module 4 to the respective work section 5 by the standardized interface 6.

Advantageously, it is proposed that the method comprises individually selecting several cartridges 2 and several drive modules 4 according to unit operations to be performed. Here it is preferred that at least a part of the cartridges 2 comprises several liquids, in particular several cell cultures. The unit operations are going to be performed on the different cell cultures. Preferably, the unit operations to be performed can be similar or of a different kind. It is preferred that the unit operations are individually adjusted according to the cell culture, on which the respective unit operation is going to be performed. For the method, respectively one of the cartridges 2 and one of the drive modules 4 are operatively coupled to each other and the coupling mechanism 7 automatically operatively couples each of the drive modules 4 to one of the work sections 5 via the standardized interface 6. The work sections 5, which are operatively coupled to one of the drive modules 4, each drive the respective drive module 4 and the respective drive module 4, which is operatively coupled to one of the cartridges 2, drives the respective cartridge 2 such that the respective unit operation is performed in the respective fluidic structure 3. The drive modules 4 may each act like a kind of translation unit, which enable flexible usage of any one of the cartridges 2 at any one of the work sections 5.

It is furthermore preferred that the method comprises an optimization routine. The optimization routine may optimize the bioprocesses, which are going to be performed. Preferably, optimization is going to be performed with regard to distances, in particular moving distances of the cartridges 2 and the drive modules 4 within the integrated bioprocessing system 1 and/or with regard to unit operations, in particular such that unit operations of the same kind are performed in neighboring work sections 5, and/or with regard to transfer of fluids, in particular such that fluid transfer, for example between different cartridges 2, can be performed efficiently.

Further, it may be the case that a drive module 4 is used, without relocating the drive module 4, for at least two cartridges 2. The optimization may therefore also be performed regarding the reuse of drive modules with multiple cartridges 2, in particular of different bioprocesses.

Another teaching which is of equal importance relates to a use of a, in particular disposable, cartridge 2 and/or a, in particular reusable, drive module 4 in an integrated bioprocessing system 1 according to one of the proposed embodiments.

## Claims

1. Integrated bioprocessing system for performing a bioprocess on a cell culture, wherein the bioprocess comprises at least one unit operation, wherein the integrated bioprocessing system (1) comprises a cartridge (2) with a fluidic structure (3) for containing a liquid, in particular the cell culture,
a drive module (4) for performing the unit operation in the fluidic structure (3) by driving the cartridge (2), wherein the cartridge (2) and the drive module (4) are individually selected according to the unit operation to be performed and operatively coupled with each other, several work sections (5), each work section (5) comprising a standardized interface (6) for operative coupling to the individually selected drive module (4), wherein each work section (5) is configured to perform the unit operation of the bioprocess by being operatively coupled to the drive module (4) and driving the drive module (4), and a coupling mechanism (7), which automatically couples the drive module (4) to one of the work sections (5) via the standardized interface (6).

2. Integrated bioprocessing system according to claim 1, **characterized in that** the integrated bioprocessing system (1) comprises several cartridges (2) each with a fluidic structure (3) for containing a liquid, in particular a cell culture, and several drive modules (4) each for performing at least one unit operation in one of the fluidic structures (3) by driving one of the cartridges (2), preferably, that at least two of the cartridges (2) and at least two of the drive modules (4) each are individually selected according to the unit operation to be performed and respectively operatively coupled with each other, wherein the at least two drive modules (4) are each automatically coupled to one of the work sections (5) via the standardized interface (6) by the coupling mechanism (7).

3. Integrated bioprocessing system according to claim 1 or 2, **characterized in that** the fluidic structure (3) of the cartridge (2) contains the liquid, in particular the cell culture, that the at least one unit operation is performed on the liquid in the fluidic structure (3), that after performing the at least one unit operation, the fluidic structure (3) is fluidically connected to a fluidic structure (3) of another cartridge (2) or of a receptacle and the liquid is transferred between the fluidically connected fluidic structures (3) of the cartridges (2).

4. Integrated bioprocessing system according to one of the preceding claims, **characterized in that** the bioprocess to be performed on the cell culture comprises a sequence of several, in particular different, unit operations, wherein, in particular, the sequence of unit operations is performed with at least two different cartridges (2) and, preferably, with at least two different drive modules (4), preferably, that during the sequence of unit operations, in particular among two consecutive unit operations, liquid, in particular the cell culture, is transferred between two of the cartridges (2).

5. Integrated bioprocessing system according to one of the preceding claims, **characterized in that** the cartridge (2) is designed as a disposable cartridge (2) and/or that the cartridge (2) is designed driveless and/or that the drive module (4) is designed as a reusable drive module (4).

6. Integrated bioprocessing system according to one of the preceding claims, **characterized in that** the cartridge (2) comprises an engagement point (8) for fluidically connecting the fluidic structure (3) of the cartridge (2) to another fluidic structure (3) of another cartridge (2) and/or a receptacle such that the liquid, in particular the cell culture, can be transferred between the fluidically connected fluidic structures (3) of the cartridges (2), preferably, that the engagement point (8) is designed as a tube engagement point (9), more preferably, that a, in particular external, tube is connectable to the tube engagement points (9).

7. Integrated bioprocessing system according to one of the preceding claims, **characterized in that** the fluidic structure (3) comprises one or more functional devices (11) for at least partially performing the unit operation and that the drive module (4) comprises a drive structure (12) for driving the cartridge (2), wherein the drive structure (12) comprises one or more drive devices (13) for driving the one or more functional devices (11).

8. Integrated bioprocessing system according to one of the preceding claims, **characterized in that** the cartridge (2) comprises at least one cartridge sensor (14) for sensing physical and/or chemical properties of the liquid, in particular of the cell culture, and/or that the drive module (4) comprises at least one module sensor (15) for sensing properties of the cartridge (2) and/or the drive module (4) and/or the properties of the liquid, in particular of the cell culture, and/or that the drive module (4) comprises a sensor control unit (16) for controlling the at least one cartridge sensor (14) and/or the at least one module sensor (15).

9. Integrated bioprocessing system according to one of the preceding claims, **characterized in that** the fluidic structure (3) comprises one or more cartridge containers (17) and/or one or more fluid transfer elements (10), preferably, that the one or more cartridge containers (17) is or are each designed as a bag, and/or, that the one or more fluid transfer elements (10) is or are each designed as a tube and/or as an active fluid transfer element (18), in particular as a valve or a pump head, preferably, that the one or more active fluid transfer elements (18), in particular the one or more valves and/or the one or more pump heads, is or are each operable by an interface actuator (19) of the standardized interface (6), and/or, that the one or more fluid transfer elements (10), in particular the one or more valves and/or the one or more pump heads, is or are each operable by a drive device (13) of the drive structure (12).

10. Integrated bioprocessing system according to one of the preceding claims, **characterized in that** the cartridge (2) comprises two or more partitions (20), which are formed by one or more partition elements (21), and/or that the cartridge (2) comprises a frame (23), wherein in particular the fluidic structure (3) is surrounded by the frame (23), and/or that the cartridge (2) comprises a housing (24), wherein the fluidic structure (3), in particular the one or more functional devices (11) and/or the one or more fluid transfer elements (10) and/or the one or more cartridge containers (17), and/or at least a part of the frame (23) is or are included in the housing (24), preferably, that the one or more partition elements (21) is or are designed such that the partitions (20) are adaptable, in particular with regard to the size of the respective partition (20), more preferably, that the partition element (21) or partition elements (21) is or are each designed as a, particularly movable, partition wall (22).

11. Integrated bioprocessing system according to one of the preceding claims, **characterized in that** the integrated bioprocessing system (1) comprises a worktop (25), wherein the standardized interfaces (6) of the work sections (5) are arranged, in particular horizontally, on the worktop (25), preferably, that the standardized interfaces (6) are arranged in a cross direction and in a longitudinal direction of the worktop (25).

12. Integrated bioprocessing system according to one of the preceding claims, **characterized in that** the standardized interface (6) of one or more work sections (5) each comprises an electrical interface (26) and/or a mechanical interface (28) and/or a data interface (27) and/or an optical interface (29) and/or a fluidic interface (30), preferably, that each of the electrical interfaces (26) comprise an electrical connector (31), particularly a power supply, and/or that each of the mechanical interfaces (28) comprise a mechanical connector (33), and/or that each of the data interfaces (27) comprise a data connector (32), particularly a digital connector or an analogue connector, and/or that each of the optical interfaces (29) comprise an optical connector (34), and/or that each of the fluidic interface (30) comprise one or more fluidic connectors (35), particularly a gas supply and/or a liquid supply, and/or, that the work sections (5) are designed each as standardized work sections (5) with the standardized interface (6).

13. Integrated bioprocessing system according to one of the preceding claims, **characterized in that** the coupling mechanism (7) comprises one or more robotic mechanisms (36), and/or, that the coupling mechanism (7) comprises one or more conveyor mechanisms (39), wherein the conveyor mechanism (39) is designed such that the cartridge (2) and/or the drive module (4) can be moved inside the integrated bioprocessing system (1), in particular in two-dimensional directions, preferably, that the one or more robotic mechanisms (36) each comprises or comprise a robotic handling mechanism (37) for handling, particularly picking and releasing, the cartridge (2) and/or the drive module (4), and, a robotic moving mechanism (38) for moving the robotic handling mechanism (37), more preferably, that the robotic moving mechanism (38) is designed such that the robotic handling mechanism (37) can be moved, in particular in three-dimensional directions, more preferably, that the robotic moving mechanism (38) is designed as a rail robotic moving mechanism (38) or as a robotic arm.

14. Integrated bioprocessing system according to one of the preceding claims, **characterized in that** the cartridge (2) comprises one or more, in particular four, robot engagement points (40), and/or that the drive module (4) comprises one or more, in particular four, robot engagement points (40), and/or that the standardized interfaces (6) of the work sections (5) each comprise or the worktop (25) comprises robot anchor points (41), preferably, that the standardized interfaces (6) each comprise four, in particular eight, robot anchor points (41).

15. Cartridge with a fluidic structure (3) for containing a liquid, in particular the cell culture, designed such that the cartridge (2) can be used in an integrated bioprocessing system (1) according to one of the claims 1 to 14.

16. Drive module for performing a unit operation in a fluidic structure (3) of a cartridge (2) by driving the cartridge (2) designed such that the drive module (4) can be used in an integrated bioprocessing system (1) according to one of the claims 1 to 14.

17. Method for performing a bioprocess on a cell culture with an integrated bioprocessing system (1), wherein the bioprocess comprises at least one unit operation and, in particular, wherein the integrated bioprocessing system (1) is designed according to one of the claims 1 to 14, wherein the method comprises individually selecting a cartridge (2) and a drive module (4) according to the unit operation to be performed and operatively coupling the cartridge (2) and the drive module (4) with each other, wherein a coupling mechanism (7) automatically operatively couples the drive module (4) to one of several work sections (5) via a standardized interface (6) of the respective work section (5), wherein the work section (5), which is operatively coupled to the drive module (4), drives the drive module (4) and the drive module (4) drives the cartridge (2) such that the unit operation is performed in the fluidic structure (3).

18. Method according to claim 17, **characterized in that** the method is performed at least in parts, in particular in total, automatically, preferably in response to an initiating computer command and/or a user command, more preferably, that the operative coupling of the consumable module and the drive module (4) is performed automatically, preferably at least partially, in particular in total, by the coupling mechanism (7).

19. Method according to claim 17 or 18, **characterized in that** the cartridge (2) and the drive module (4) are operatively coupled before operative coupling of the drive module (4) to the respective work section (5) by the standardized interface (6) or that the cartridge (2) and the drive module (4) are operatively coupled after operative coupling of the drive module (4) to the respective work section (5) by the standardized interface (6) or that the cartridge (2) and the drive module (4) are operatively coupled at least partially simultaneously to operational coupling of the drive module (4) to the respective work section (5) by the standardized interface (6).

20. Method according to one of the claims 17 to 19, **characterized in that** the method comprises
individually selecting several cartridges (2) and several drive modules (4) according to unit operations to be performed, wherein the unit operations to be performed can be similar or different unit operations, and operatively coupling respectively one of the cartridges (2) and one of the drive modules (4) to each other, wherein the coupling mechanism (7) automatically operatively couples each of the drive modules (4) to one of the work sections (5) via the standardized interface (6), wherein the work sections (5), which are operatively coupled to one of the drive modules (4), each drive the respective drive module (4) and the respective drive module (4), which is operatively coupled to one of the cartridges (2), drives the respective cartridge (2) such that the respective unit operation is performed in the respective fluidic structure (3).

21. Use of a, in particular disposable, cartridge (2) and/or a, in particular reusable, drive module (4) in an integrated bioprocessing system (1) according to one of the claims 1 to 14.
